# EUROPEAN PATENT SPECIFICATION

(11) **EP 1 507 571 B1**
(45) Date of publication and mention of the grant of the patent: **03.01.2007**
(21) Application number: 03720843.6
(22) Date of filing: 15.05.2003
(51) Int. Cl.: A61M 29/02, A61M 25/10, A61M 25/00

(54) **DILATOR FOR BODY PASSAGEWAY**
DILATATOR FÜR EINEN KÖRPERKANAL
DILATATEUR SERVANT A ELARGIR UN PASSAGE VERS UN CORPS

(30) Priority: 15.05.2002 IL 14968702
(43) Date of publication of application: 23.02.2005
(73) Proprietor: Medilator, Migdal Emek 23100 (IL)
(72) Inventor: CONDREA, Alexander, 34792 Haifa (IL); VADASZ, Arie, 32446 Haifa (IL)
(74) Representative: Casey, Lindsay Joseph
(86) International application number: PCT/IL2003/000400
(87) International publication number: WO 2003/097154

(56) References cited:
- EP-A- 0 274 411
- EP-A- 1 051 990
- WO-A-01/12255
- WO-A-01/89619
- US-A- 5 152 776
- US-A- 5 348 538
- US-A- 5 449 371
- US-A- 5 624 399
- US-A- 5 645 789
- US-A- 5 769 817
- US-A- 6 120 523

## Description

### FIELD OF THE INVENTION

The present invention relates to medical devices and more specifically to such devices for dilating a body passageway.

### BACKGROUND OF THE INVENTION

It is often necessary to dilate a body passageway, such as a blood vessel, urethra, or cervix. In the case of the cervix, for example, it is sometimes necessary to dilate the cervix in order to allow access of surgical instruments into the uterine cavity during a medical procedure. Such procedures include induced abortion, completion of a spontaneous abortion, and operative hysteroscopy.

The human cervix is a tubular structure between 2 to 5 cm in length. The cervical canal is usually closed, but a catheter having a diameter of 2 to 3 mm can be introduced into the cervical canal with only minor discomfort to a female patient in the absence of any anesthesia. It is usually required to dilate the cervix to a diameter between 10-12 mm.

One known method for dilating the cervix uses Hegar dilators. These are essentially metal rods. A dilator of a relative small caliber is inserted into the cervix to achieve a small dilation. The rod is removed, and a rod of slightly larger caliber is inserted into the cervix. This rod is then removed, and the process repeated, each time with a rod of larger caliber, until the desired dilation has been achieved. This procedure requires some form of anesthesia, and may damage the cervix.

U.S. Patent No. 4,624,258 to Stubbs discloses a hygrometric dilator comprising an insertion body containing the vegetative stalks of Laminaria japonica. The insertion body is inserted into the cervix, whereupon the Laminaria expands as it absorbs fluids present in the cervix, causing the cervix to dilate.

WO 81/01098 discloses a device for inflating a cervix comprising an inflatable latex balloon. The balloon is enclosed in a sleeve formed from an inelastic material in order to allow the balloon to withstand high pressures.

US Patent No. 4,137,222 to Leininger discloses an inflatable device having an inflatable balloon with an enlarged bulbous portion at one end. When the balloon is inflated, the enlarged portion expands inside the uterus while the remainder of the balloon inflates in the cervix. The enlarged portion prevents the balloon from being expelled out of the cervix.

United States Patent Specification No US-A-6 120 523 discloses discloses a focal balloon having at least one reference zone and a focal zone. In one embodiment, the reference zone and focal zone are inflatable to a first generally cylindrical profile at a first pressure. At a second, greater pressure, the focal section expands to a second, greater diameter, while the reference zone remains substantially at the first diameter. Multiple lobed and drug delivery embodiments are also disclosed..

United States Patent Specification No US-A-5 645 789 discloses a semi-compliant PET balloon and a method for manufacturing the balloon are disclosed. The method for manufacturing the semi-compliant PET balloon includes the steps of varying the temperature and inflation pressure on a tube of PET material in an ordered sequence to i) form the balloon, ii) thin the walls of the balloon, ii) size the balloon and then, iv) crystallize the PET material of the balloon.

United States Patent Specification No US-A-5 769 817 discloses an expander member for a balloon catheter to be used in stent delivery applications comprises a double-walled tubular member that is adapted for attachment to a distal end portion of an elongated, flexible, tubular catheter body. The outer wall is preferably a polyamide, such as Nylon-12, and the inner wall is PET. By properly controlling the percentage of the polyamide relative to the PET content, the distension characteristics of the resulting expander member can be tailored to fall in a range less than 15 percent. The outer Nylon-12 layer provides the expander member with high abrasion resistance. By appropriately temperature annealing the expander member, any tendency toward winging upon deflation thereof is reduced.

United States Patent Specification No US-A-5 624 399 discloses a catheter for non-surgical entry into the cervical canal or the uterus, comprising an elongated tubular catheter body. An inflatable balloon manufactured from a polyurethane material is disposed proximal to the distal end of the catheter body. The polyurethane inflatable balloon can be progressively inflated into an elliptical shape which enables the balloon to be used within the cervical canal. Additional inflation pressure inflates the polyurethane inflatable balloon to a spherical shape which enables the balloon to be used within the uterus.

### SUMMARY OF THE INVENTION

According to an aspect of the present invention, there is provided a device for dilating a body passageway as specified in claim 1.

While the device will be described in relation to dilating a cervix, it should be understood that the device of the invention may be used to dilate other body cavities such as a urethra or blood vessel.

The device of the invention comprises a hollow shaft having a distal end and a proximal end. The shaft typically has a diameter of about 1 to2 mm. At the distal end of the shaft is an inflatable balloon. A pressurized fluid is made to flow in the lumen of the hollow shaft from the proximal end to the distal end so as to inflate the balloon. The balloon has at least a portion that is cylindrical in shape when inflated.

The balloon is formed from a flexible essentially unstretchable material. The material of the balloon is further selected so that the inflated balloon can withstand pressures of up to 10 bar, more preferably 20 bar, and still more preferably, 30 bar. The wall of the balloon is sufficiently thin so that when the uninflated balloon is collapsed onto the shaft, the diameter of the balloon on the shaft is at most 35%, more preferably 30%, even more preferably 25 % of the diameter of the inflated cylindrical portion of the balloon. The thickness of the wall of the balloon is preferably less than 0.1 mm, more preferably less than 0.08 mm, and still more preferably around 0.05 mm.

For example, the inventors have found that a cylindrical balloon having an inflated diameter of 12 mm formed from polyethylene perephthalate (PET) or polyamide and having a wall thickness of 0.05 mm can withstand pressures up to at least 14 bar. Since the maximum pressure that a cylindrical balloon can withstand is proportional to its wall thickness, a balloon of this material and inflated diameter having a wall thickness of 0.10 mm can withstand pressures up to 28 bar. Similarly, a balloon of this material and inflated diameter having a wall thickness of 0.20 mm can withstand pressures up to 42 bar. A cylindrical balloon having an inflated diameter of up to 10 mm and a wall thickness up to 0.2 mm, will have, when collapsed onto a 1 mm diameter shaft, a diameter less than 3 mm.

The proximal end of the shaft is adapted to be connected to a source of a pressurized fluid. The fluid is preferably an incompressible fluid such as water or saline. The fluid may be delivered from the proximal end of the shaft to the distal end of the shaft and into the balloon by any means of pressurizing the fluid. For example, the fluid may be loaded into a syringe that is placed in fluid communication with the lumen of the shaft. The fluid is then manually pressurized by displacing the piston of the syringe.

In a preferred embodiment, the fluid is pressurized by an electrical pump that delivers the fluid through the shaft to the balloon at a predetermined flow rate. The flow rate is selected to be slow enough so as to inflate the balloon at a rate that does not cause severe pain to the patient. The device may thus be used without anesthetizing the patient.

Since the balloon is formed from an unstretchable material, when an incompressible fluid is used to inflate the balloon, the volume of the balloon may be determined at any time from the amount of fluid delivered to the balloon. In particular, the maximum volume of the balloon is obtained when that volume of fluid has been delivered to the balloon.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Fig. 1** shows a dilator having a balloon, in accordance with the invention;
**Fig. 2** shows the dilator of Fig. 1 with the balloon in an uninflated state after insertion into a cervix,
**Fig. 3** shows the dilator of Fig. 1 after insertion into a cervix with the balloon in a partially inflated state;
**Fig. 4** shows the dilator of Fig. 1 after insertion into a cervix with the balloon in an inflated state;
**Fig. 5** shows a dilator having two balloons;
**Fig. 6** shows a dilator having two balloons and a shaft with two lumens;
**Fig. 7** shows a dilator having two balloons; and
**Figs. 8 to 11** show deployment of the dilator of Fig. 7.

### DETAILED DESCRIPTION OF THE INVENTION

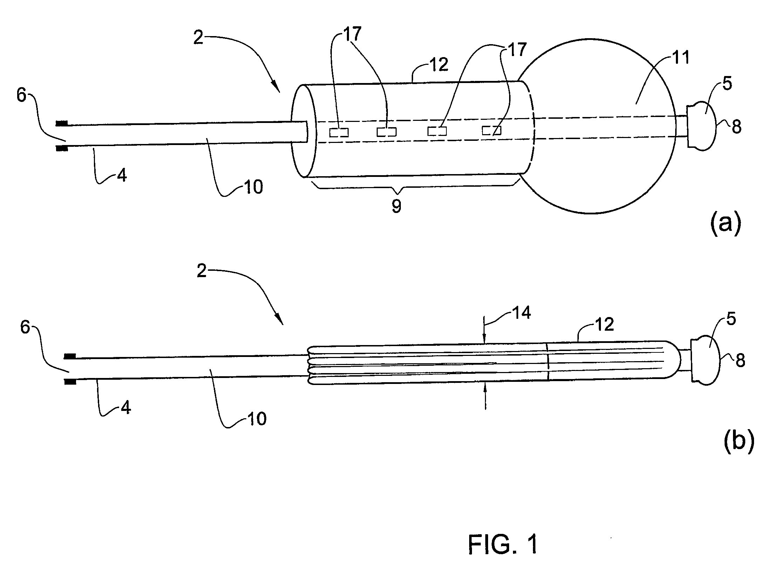

Fig. 1 shows a device for dilating a body passageway, in accordance with the invention. The device generally indicted by **2,** includes an elongated shaft **4** having a proximal end **6** and a distal end **8.** The shaft **4** has an outer diameter of 1 to 2 mm and encloses a lumen **10.** The shaft is formed from a flexurally resilient material such as plastic, stainless steel, or polyurethane. A blunt cap **5** at the distal end **8** of the shaft **4** prevents damage to tissues during insertion.

An inflatable balloon **12** located at the distal end of the shaft **4** is formed from a flexible material that is essentially unstretchable. The balloon **12** is shown in Fig. 1a in its inflated state. The inflated balloon **12** has a generally cylindrical shaped body portion **9** with a length of about 6 cm and an inflated diameter of about 1.2 cm. The cylindrical portion is coaxial with the shaft and has an annular cross-section. The balloon terminates in an ellipsoidal portion **11** having an axis of about 15 mm perpendicular to the axis of the shaft **4.**

Fig. 1b shows the device **2** with the balloon **12** in its uninflated state. The balloon **12** has been collapsed and wrapped around the shaft **4.** The wall of the balloon **12 has** a thickness of about 0.02-0.05 mm, so that the diameter **14** of the collapsed balloon on the shaft **11** does not exceed 3 mm.

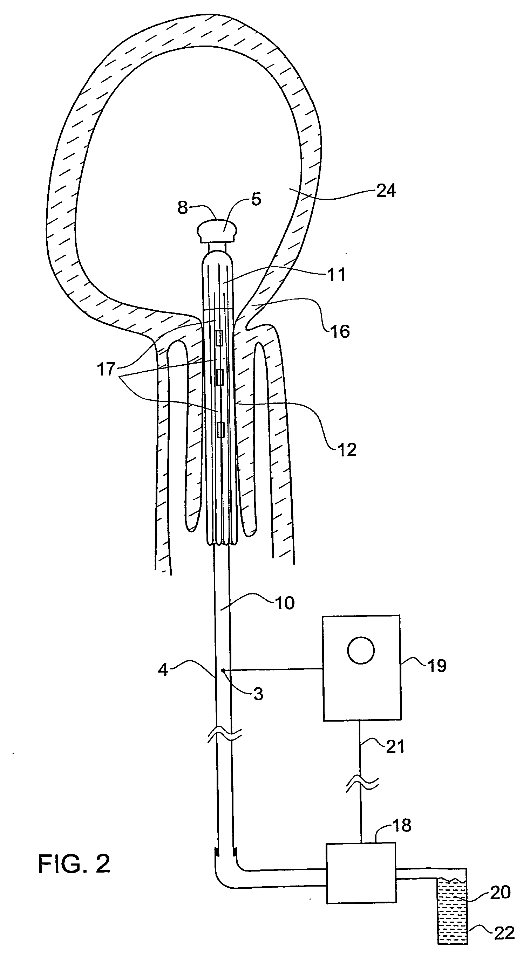

The balloon **12** is delivered to the passageway to be dilated with the uninflated balloon collapsed on the shaft **4.** Fig. 2 shows the device **2** after delivery of the balloon **12** in its uninflated state to a cervix **16.** The distal end **8** and the ellipsoidal portion **11** are located in the uterus **24.** An electric pump **18** is used to deliver an incompressible fluid **20** in a reservoir **22** to the balloon **12.** The fluid flows from the pump **18** through the lumen **10** of the shaft **4** through openings **17** in the distal end of the shaft **4** into the balloon **12.** The pump **18** is may be programmable so as to deliver the fluid **20** at a rate that builds up pressure in the balloon **12** sufficiently slow so as to prevent excessive pain to the patient in the absence of anesthesia. Alternatively, the pump **18** may have a selectable flow rate by means of a flow rate selector **19.** The flow rate selector **19** may be located remote from the pump **18,** for example, by means of a cable **21,** or by a remote control (not shown). A remotely positionable flow rate selector **19** may be controlled by the patient so that the patient may select a flow rate that does not cause excessive pain. The pump **18** may be connected to a pressure gauge **3** that measures the fluid pressure inside the balloon **12** during inflation. In this case, the pump **18** may be programmed to deliver fluid to the balloon **12** so as to obtain a predetermined rate of increase in pressure. The pump **18** may further be programmable to discontinue delivery of the fluid **20** to the balloon when the volume of fluid **20** delivered to the balloon **12** is equal to the maximum capacity of the balloon **4.**

As the balloon **12** is inflated, the ellipsoidal portion **11** of the balloon inflates before the cylindrical portion **9,** even though the thickness of the wall of the balloon is uniform throughout the cylindrical and ellipsoidal portions of the balloon **12.** This is because the ellipsoidal portion **11,** being located in the uterus **24,** is subjected to a lower external pressure than the cylindrical portion **11** in the cervix **16.**

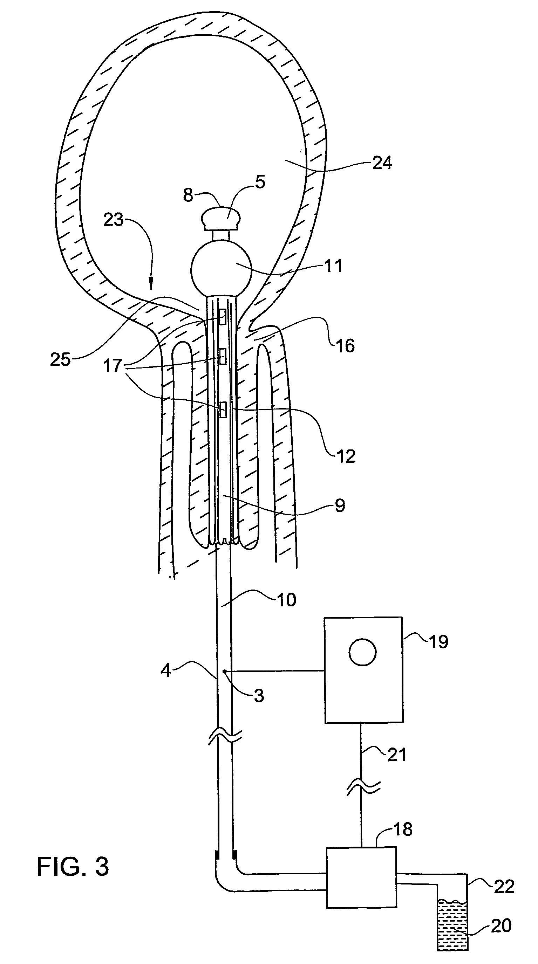

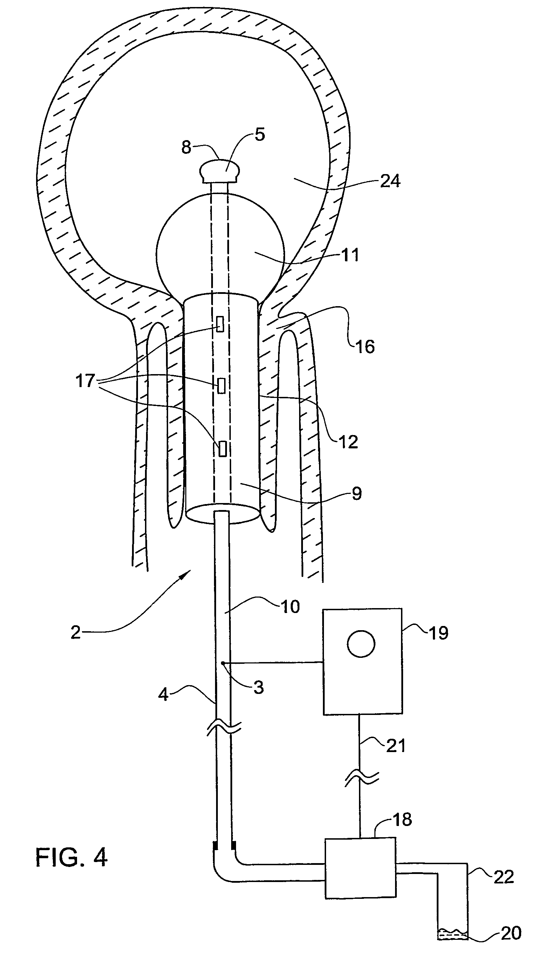

Fig. 3 shows the device **2** after partial inflation of the balloon **12,** showing the ellipsoidal portion **11** is almost completely inflated, and the cylindrical portion **9** only partially inflated. At this point, the balloon **12** is moved proximally in the direction of the arrow **23,** so as to lodge the ellipsoidal portion **11** in the internal os **25.** This ensures that the cylindrical portion is appropriately positioned in the cervix. Inflation of the balloon **12** continues until the balloon **12** has attained its maximum volume, at which time the cylindrical portion **9** is completely distended and has a diameter of at least 10 cm and preferably 12 cm. The pressure in the balloon when dilation of cervix **16** is complete may exceed 10 bar, 20 bar, 30 bar or 40 bar. Fig. 4 shows the dilator **2** with the balloon **4** completely inflated and the ellipsoidal portion **11** of the balloon 4 lodged in the internal os **25.** The balloon **12** is now deflated and the device **2** is withdrawn from the body.

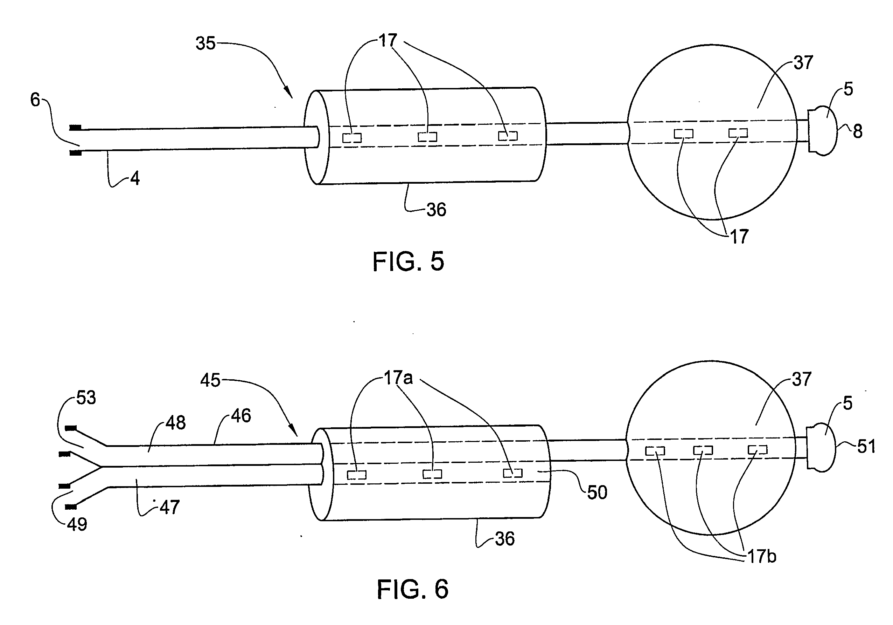

Fig 5 shows another device, generally indicated by **35.** The device **35** has features in common with the device **2,** and similar components are identified with the same numeral without further comment. The embodiment **35** has two balloons **36** and **37,** that are shown in their inflated state in Fig. 5. The balloon **36** has a cylindrical shape when inflated, and has dimensions similar to those of the cylindrical portion **9** of the balloon **12.** The balloon **37** is ellipsoidal in shape and has dimensions similar to those of the ellipsoidal portion **11** of the balloon **12.** The device **35** is used similarly to the device **2** as shown in Figs. 2 to 4. Both balloons **36** and **37** are in fluid communication with the shaft **11.** As explained above with reference to the ellipsoidal portion **11** of the balloon **12,** the ellipsoidal balloon **37** will inflate before the cylindrical balloon **36.** When the ellipsoidal balloon **37** is inflated, the shaft **4** is moved proximally until the ellipsoidal balloon **37** is lodged in the internal os. The shaft **4** is then moved distally a distance about equal to the spacing between the two balloons **36** and **37** along the shaft, so as to position the cylindrical balloon **36** in the cervix.

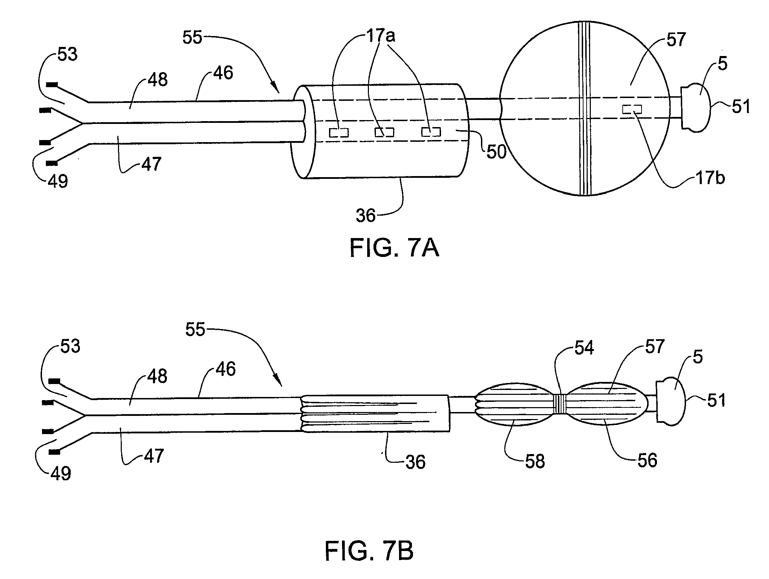

Fig 6 shows another device generally indicated by **45.** The device **45** has features in common with the device **35,** and similar components are identified with the same numeral without further comment. In particular, the embodiment **45** has two balloons **36** and **37,** that are shown in their inflated state in Fig.6. The balloon **36** has a cylindrical shape when inflated, and has dimensions similar to those of the cylindrical portion **9** of the balloon **12.** The balloon **37** is ellipsoidal in shape and has dimensions similar to those of the ellipsoidal portion **11** of the balloon **12.** In contrast to the device **35,** the device **45** has a shaft **46** containing a first lumen **47** and a second lumen **48.** The lumen **47** has a proximal end **49** and a distal end **50** located in the cylindrical balloon **36.** Fluid is delivered from the lumen **47** into the cylindrical balloon **36** through openings **17a** in the shaft **46.** The lumen **48** has a proximal end **53** and a distal end **51** located distal to the spherical balloon **36.** Fluid is delivered from the lumen **48** into the spherical balloon **37** through openings **17b** in the shaft **46.** Each of the lumens **47** and **48** may be connected to a pump **18** and reservoir **20** (as shown in Figs. 2 to 4) so that the balloons **36** and **37** may be inflated independently. The device **45** is used similarly to the device **2** as shown in Figs. 2 to 4. In particular, the ellipsoidal balloon **37** can be inflated before the cylindrical balloon **36.** This is done in order to position the cylindrical balloon **36** in the cervix as described above with reference to the embodiment of Fig. 5.

Fig 7 shows another device generally indicated by **55.** The device **55** has features in common with the device **45** of Fig. 6, and similar components are identified with the same numeral without further comment. The embodiment **55** has two balloons **36** and **57,** that are shown in their inflated state in Fig. 7a, and in their uninflated state in Fig. 7b. The balloon **36** has a cylindrical shape when inflated, and has dimensions similar to those of the cylindrical portion **9** of the balloon **12.** The balloon **57** is ellipsoidal in shape when inflated and has inflated dimensions similar to those of the ellipsoidal portion **11** of the balloon **12.** The balloon **57** when uninflated is constricted around the shaft **46,** at an elastic constriction site **54** so as to form two separately inflatable compartments in the uninflated balloon **57.** As explained in detail below, pressurized fluid in the lumen **48** is released only into a distal compartment **56.** When fluid pressure in the distal compartment exceeds a predetermined value, the fluid forces passes from the distal compartment **56** into a proximal compartment **58,** as explained in detail below.

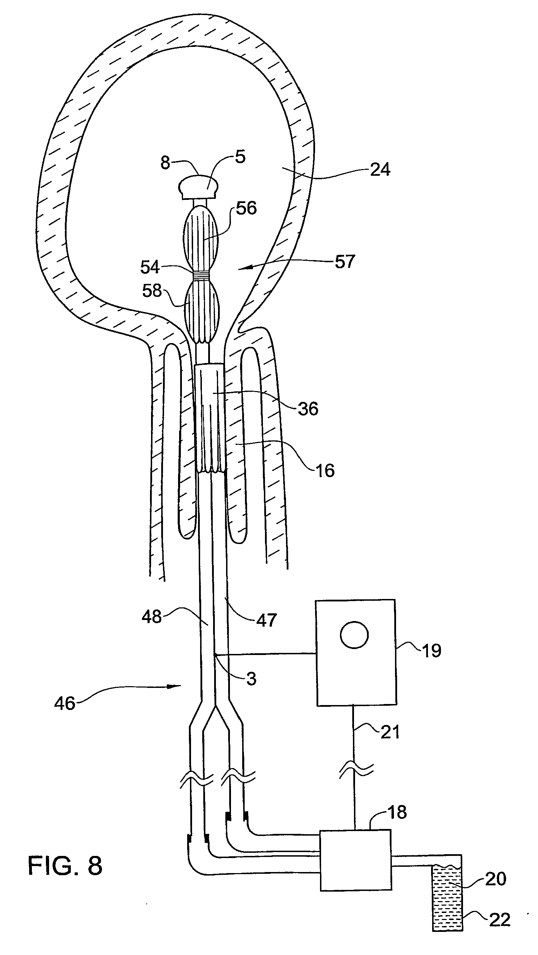

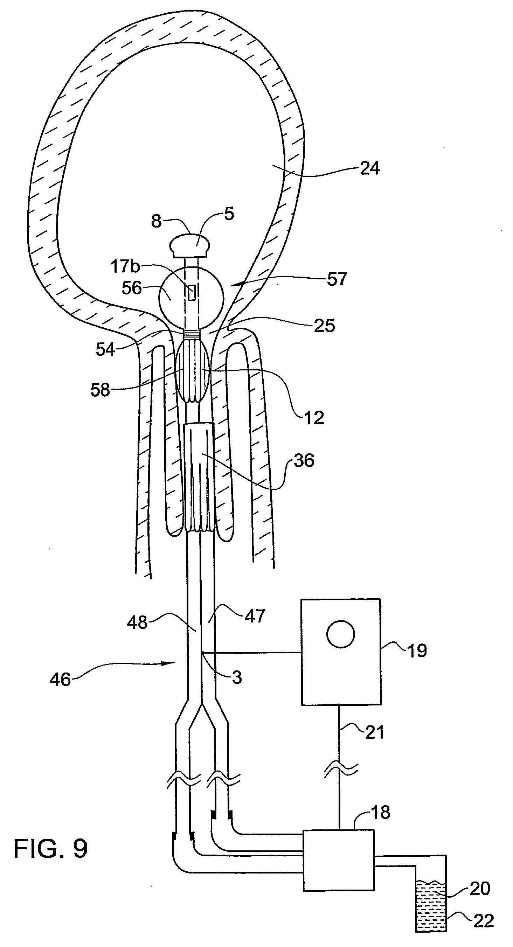

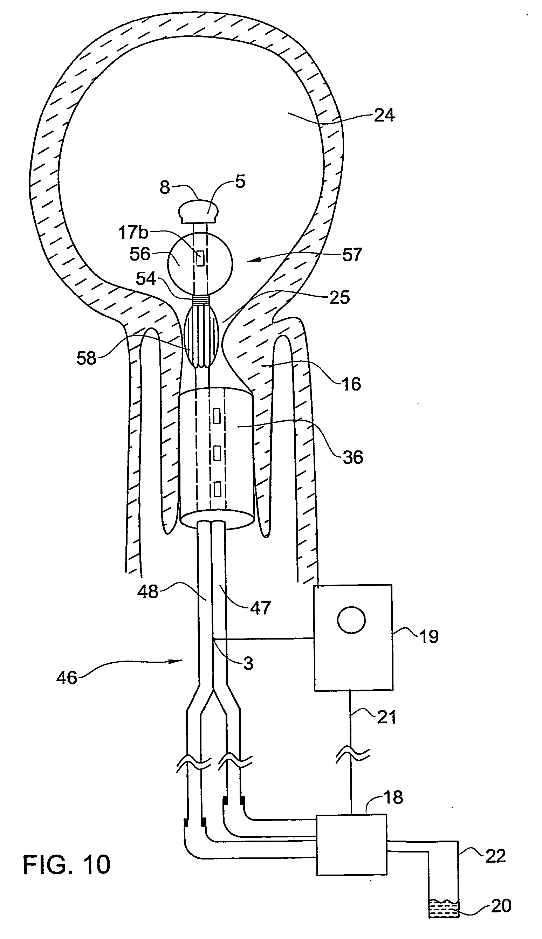

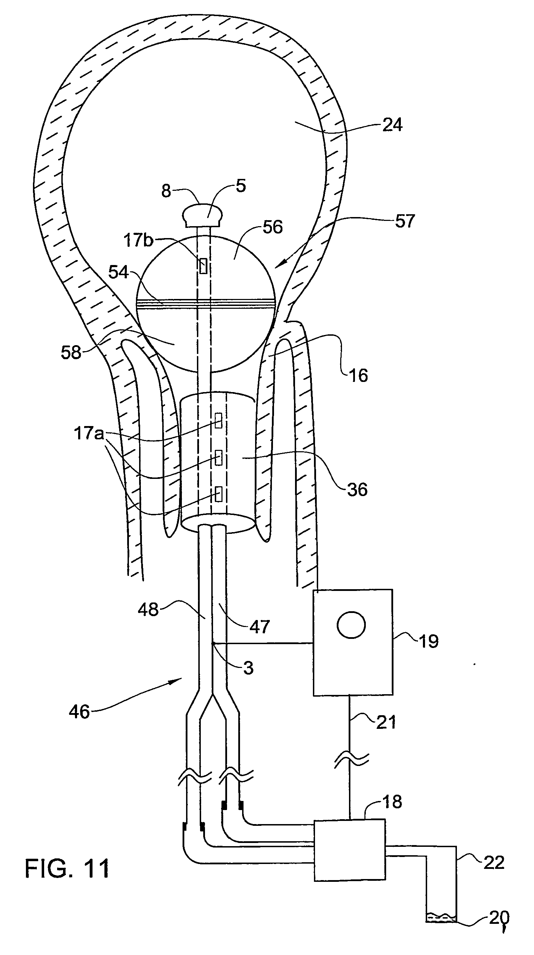

Figs. 8 to 10 show deployment of the device **55.** As shown in Fig. 8, the balloon **36** is delivered to the cervix **16** and the balloon **57** is delivered to the uterus **24,** with the balloons **36** and **57** in their uninflated state and collapsed on the shaft **46.** Fluid is now delivered through the lumen **48** of the shaft **46** and the opening **17b** into the distal compartment **56** of the balloon **57,** until inflation of the distal compartment **56** is complete, as shown in Fig. 9. Due to the elastic constriction area **54,** the proximal compartment **58** of the balloon **57** does not inflate at this point. The inflated distal compartment **56** is then lodged in the internal os **25,** so as to ensure that the cylindrical balloon **36** is properly positioned in the cervix **16.** The proximal compartment **58** is now located in the distal termination of the cervix **16.** Now pressurized fluid is delivered to the cylindrical balloon via the lumen **48** of the shaft **46.** Fig. 10 shows the device **55** after inflation of the cylindrical balloon **36** is complete. Now additional fluid is delivered to the distal compartment **56** of the ellipsoidal balloon. When the fluid pressure in the distal compartment **56** exceeds a predetermined threshold, the elastic constriction area **54** expands allowing fluid to flow into the proximal compartment. Fig. 11 shows the device with the balloon **57** completely inflated. As the proximal compartment expands **58,** it causes the distal terminal segment of the cervix **16** to expand. A spherical balloon can withstand a pressure that is twice that of a cylindrical balloon made of the same material and having the same wall thickness. Thus, the spherical balloon can deliver a pressure to the distal terminal portion of the cervix (where the resistance to dilation is greatest) that is about twice the pressure that can be delivered to the cervix by the cylindrical balloon

## Claims

1. A device (2) for dilating a body passageway comprising:
(a) an elongated hollow shaft (4) having a lumen (10), a proximal end (6) and a distal end (8), and
(b) a balloon (12) at the distal end (8) of the shaft (4) in fluid communication with the lumen (10) of the shaft (4, 47) **characterised in that**:
the balloon has a cylindrical portion (9) and an ellipsoidal portion (11) distal to the cylindrical portion (9).

2. A device (2) as claimed in claim 1 wherein the balloon (12) is formed from polyethylene perephthalate (PET) or polyamide.

3. A device as claimed in claim 1 or claim 2 further comprising a reservoir (20) for containing a fluid (20).

4. A device as claimed in claim 3 wherein the reservoir (20) contains an incompressible fluid (20).

5. A device as claimed in claim 3 further comprising a pump (18) for delivering fluid (20) from the reservoir (20) to the balloon (12).

6. A device as claimed in claim 5 wherein the pump (18) is configured to deliver fluid (20) from the reservoir (20) to the balloon (12) at a predetermined or selectable flow rate.

7. A device as claimed in claim 5 further comprising a pressure gauge measuring fluid pressure in the balloon (12) and wherein the pump (18) is configured to deliver fluid (20) from the reservoir (20) to the balloon (12) so as to achieve a predetermined or selectable rate of increase of pressure in the balloon (12).

8. A device as claimed in claim 5 wherein the pump (18) has a flow rate that is remotely controlled.

9. A device as claimed in any of claims 1-8 wherein the balloon (12) contains an area (54) that is elastically constricted onto the shaft (4) when the balloon (12) is uninflated so as to form a distal compartment (56) and a proximal compartment (58), the distal compartment (56) being in fluid communication with a second lumen (48) and the proximal compartment (58) not being in fluid communication with the second lumen (48) when fluid pressure in the distal compartment (56) is below a predetermined threshold.

10. A device as claimed in any of claims 1-9 for dilating a cervix, urethra or blood vessel.

## Patentansprüche

1. Vorrichtung (2) zum Weiten eines Körperdurchgangs, die Folgendes umfasst:
(a) einen langen hohlen Schaft (4), der ein Lumen (10), ein proximales Ende (6) und ein distales Ende (8) aufweist, und
(b) einen Ballon (12) an dem distalen Ende (8) des Schafts (4), der mit dem Lumen (10) des Schafts (4, 47) in Fluidkommunikation steht, **dadurch gekennzeichnet, dass**:
der Ballon einen zylindrischen Abschnitt (9) und einen elliptischen Abschnitt (11) distal von dem zylindrischen Abschnitt (9) aufweist.

2. Vorrichtung (2) nach Anspruch 1, wobei der Ballon (12) aus Polyethylenterephthalat (PET) oder Polyamid gebildet ist.

3. Vorrichtung nach Anspruch 1 oder Anspruch 2, die des Weiteren einen Behälter (20) umfasst, der ein Fluid (20) enthält.

4. Vorrichtung nach Anspruch 3, wobei der Behälter (20) ein nicht komprimierbares Fluid (20) enthält.

5. Vorrichtung nach Anspruch 3, die des Weiteren eine Pumpe (18) zur Zufuhr von Fluid (20) von dem Behälter (20) zu dem Ballon (12) umfasst.

6. Vorrichtung nach Anspruch 5, wobei die Pumpe (18) so konfiguriert ist, dass sie dem Ballon (12) Fluid (20) von dem Behälter (20) bei einer vorherbestimmten oder wählbaren Strömungsrate zuführt.

7. Vorrichtung nach Anspruch 5, die des Weiteren einen Druckmesser umfasst, der den Fluiddruck in dem Ballon (12) misst, und wobei die Pumpe (18) so konfiguriert ist, dass sie dem Ballon (12) Fluid (20) so von dem Behälter (20) zuführt, dass eine vorherbestimmte oder wählbare Druckzunahmerate in dem Ballon (12) erreicht wird.

8. Vorrichtung nach Anspruch 5, wobei die Pumpe (18) eine Strömungsrate aufweist, die ferngesteuert wird.

9. Vorrichtung nach einem der Ansprüche 1 bis 8, wobei der Ballon (12) einen Bereich (54) enthält, der elastisch an den Schaft (4) gezogen ist, wenn der Ballon (12) nicht aufgepumpt ist, um eine distale Kammer (56) und eine proximale Kammer (58) zu bilden, wobei die distale Kammer (56) mit einem zweiten Lumen (48) in Fluidkommunikation steht und die proximale Kammer (58) nicht mit dem zweiten Lumen (48) in Fluidkommunikation steht, wenn der Fluiddruck in der distalen Kammer (56) unter einem vorherbestimmten Grenzwert liegt.

10. Vorrichtung nach einem der Ansprüche 1 bis 9 zum Weiten eines Gebärmutterhalses, einer Harnröhre oder eines Blutgefäßes.

## Revendications

1. Dispositif (2) pour dilater un passage anatomique comprenant :
(a) une tige creuse allongée (4) ayant une lumière (10), une extrémité proximale (6) et une extrémité distale (8), et
(b) un ballon (12) à l'extrémité distale (8) de la tige (4) en communication fluidique avec la lumière (10) de la tige (4, 47) **caractérisé en ce que** :
ce ballon a une portion cylindrique (9) et une portion ellipsoïdale (11) distale par rapport à la portion cylindrique (9).

2. Dispositif (2) tel que revendiqué dans la revendication 1, le ballon (12) étant formé à partir de polyéthylène téréphtalate (PET) ou de polyamide.

3. Dispositif tel que revendiqué dans la revendication 1 ou la revendication 2, comprenant également un réservoir (20) pour contenir un fluide (20).

4. Dispositif tel que revendiqué dans la revendication 3, le réservoir (20) contenant un fluide incompressible (20).

5. Dispositif tel que revendiqué dans la revendication 3, comprenant également une pompe (18) pour refouler du fluide (20) du réservoir (20) au ballon (12).

6. Dispositif tel que revendiqué dans la revendication 5, la pompe (18) étant configurée pour refouler du fluide (20) du réservoir (20) au ballon (12) à un débit prédéterminé ou qui peut être sélectionné.

7. Dispositif tel que revendiqué dans la revendication 5, comprenant également une jauge de pression mesurant la pression du fluide dans le ballon (12), et dans lequel la pompe (18) est configurée pour refouler du fluide (20) du réservoir (20) au ballon (12) de façon à obtenir dans le ballon (12) une vitesse d'augmentation de pression prédéterminée ou qui peut être sélectionnée.

8. Dispositif tel que revendiqué dans la revendication 5, la pompe (18) ayant un débit qui est commandé à distance.

9. Dispositif tel que revendiqué dans l'une quelconque des revendications 1 à 8, le ballon (12) contenant une partie (54) qui est étranglée élastiquement sur la tige (4) lorsque le ballon (12) n'est pas gonflé de façon à former un compartiment distal (56) et un compartiment proximal (58), le compartiment distal (56) étant un communication fluidique avec une deuxième lumière (48) et le compartiment proximal (58) n'étant pas en communication fluidique avec la deuxième lumière (48) lorsque la pression du fluide dans le compartiment distal (56) est en dessous d'un seuil prédéterminé.

10. Dispositif tel que revendiqué dans l'une quelconque des revendications 1 à 9 pour dilater un col, un urètre ou un vaisseau sanguin.
